# EUROPEAN PATENT APPLICATION

(11) **EP 4 800 116 A1**
(43) Date of publication of application: **02.09.2026**
(21) Application number: 25195011.9
(22) Date of filing: 08.08.2025
(51) Int. Cl.: C12P 7/40, C12P 7/54, C12P 39/00, C12N 1/20

(54) **METHOD FOR SYNTHESIZING CAPROIC ACID BY CARBON CHAIN EXTENSION BASED ON TWO-STAGE ANAEROBIC FERMENTATION**

(30) Priority: 26.02.2025 CN 202510220365
(71) Applicant: Taiyuan University of Technology, Taiyuan, Shanxi 030024 (CN)
(72) Inventor: ZHOU, Aijuan, Jinzhong, 030600 (CN); LIU, Zhihong, Jinzhong, 030600 (CN); KANG, Kaiming, Jinzhong, 030600 (CN); LI, Dengfei, Jinzhong, 030600 (CN); YUE, Xiuping, Jinzhong, 030600 (CN)
(74) Representative: Bayramoglu et al.

(57) **Abstract**

The present invention belongs to the technical field of sewage treatment, and discloses a method for synthesizing caproic acid by carbon chain extension based on two-stage anaerobic fermentation. The method according to the present invention includes the following steps: performing alkali pretreatment on concentrated sludge; performing first anaerobic fermentation on the concentrated sludge subjected to alkali pretreatment to obtain a fermentation product; and mixing the fermentation product, caproic acid-producing bacteria, an electron donor, nutrients and a first methane inhibitor, and performing second anaerobic fermentation to obtain the caproic acid. The present invention is more beneficial to carbon chain extension reaction by two-stage anaerobic fermentation. Different from a one-stage anaerobic fermentation system that features a slow reaction process due to the lack of short-chain fatty acids, the two-stage anaerobic fermentation process generates more caproic acid. In addition, the alkali pretreatment effectively destroys the structure of extracellular polymeric substances, which can release more dissolved organic matter, increase the concentration of acetic acid, provide more organic substrate, and improve the synthesis of caproic acid.

## Description

### TECHNICAL FIELD

The present invention relates to the technical field of sewage treatment, and in particular, to a method for synthesizing caproic acid by carbon chain extension based on two-stage anaerobic fermentation.

### BACKGROUND

With the increasing discharge of industrial wastewater and domestic sewage, 90% of sewage treatment processes currently adopt an activated sludge process, resulting in a continuous increase in the amount of excess sludge. The sludge contains rich organic matter and nutrients, of which organic carbon compounds account for about 60% of the dry weight; followed by organic compounds containing nitrogen and phosphorus, which account for 3.5% and 2.0% of the dry weight, respectively. Therefore, promoting the resource utilization of sludge is a very promising direction, which is in line with the technical concept of "harmless resource treatment and disposal of sludge". Considering the sustainable development of economy and environment, in recent years, researchers have focused more on the production of renewable biochemicals from waste activated sludge.

Based on the characteristics of high energy recovery and low environmental impact of traditional anaerobic digestion processes, researchers find that the biochemical conversion of sludge as organic matter into high value-added products has wide prospects, especially in the synthesis of caproic acid by anaerobic fermentation of sludge. However, the current solution has problems of low yield and difficulty in breaking the sludge cell wall when synthesizing caproic acid by anaerobic fermentation of sludge.

### SUMMARY

An objective of the present invention is to provide a method for synthesizing caproic acid by carbon chain extension based on two-stage anaerobic fermentation, which solves the problems of low caproic acid yield and difficult sludge cell wall breaking of the existing fermentation solution.

To achieve the above objective, the present invention provides the following technical solutions.

The present invention provides a method for synthesizing caproic acid by carbon chain extension based on two-stage anaerobic fermentation, which includes the following steps:
performing alkali pretreatment on concentrated sludge; performing first anaerobic fermentation on the concentrated sludge subjected to alkali pretreatment to obtain a fermentation product; and
mixing the fermentation product, caproic acid-producing bacteria, an electron donor, a culture substrate and a first methane inhibitor, and performing second anaerobic fermentation to obtain the caproic acid.

Preferably, in the method for synthesizing caproic acid by carbon chain extension based on two-stage anaerobic fermentation, a pH value of the concentrated sludge is 7.33-7.37, a total suspended solids concentration of the concentrated sludge is 31.81-31.85 g/L, and a volatile suspended solids concentration of the concentrated sludge is 12.03-12.07 g/L.

Preferably, in the method for synthesizing caproic acid by carbon chain extension based on two-stage anaerobic fermentation, the alkali pretreatment conditions include: a pH value of 10-14, a temperature of 110-130 °C, and a reaction time of 10-30 min.

Preferably, in the method for synthesizing caproic acid by carbon chain extension based on two-stage anaerobic fermentation, the first anaerobic fermentation conditions include: an initial pH value of 5-7, a temperature of 30-40 °C, a rotation speed of 100-200 rpm, and a reaction time of 3-4 days.

Preferably, in the method for synthesizing caproic acid by carbon chain extension based on two-stage anaerobic fermentation, the caproic acid-producing bacteria include one or more of Clostridium_sensu_stricto, Romboutsia, and Terrisporobacter; and
a mass ratio of the concentrated sludge to the caproic acid-producing bacteria is 1-2:9-12.

Preferably, in the method for synthesizing caproic acid by carbon chain extension based on two-stage anaerobic fermentation, the caproic acid-producing bacteria are acclimatized before use;
the acclimatization process comprises the following steps: mixing the caproic acid-producing bacteria, a chain extension substrate, a culture medium and a second methane inhibitor under an anaerobic condition for culturing; and
the culture conditions comprise: a pH value of 4-8, a temperature of 20-50 °C, a rotation speed of 100-200 rpm, and a culture time of 2-7 days.

Preferably, in the method for synthesizing caproic acid by carbon chain extension based on two-stage anaerobic fermentation, the electron donor includes ethanol and/or lactic acid; and
a molar ratio of an electron acceptor to the electron donor in the fermentation product is 1:3-5.

Preferably, in the method for synthesizing caproic acid by carbon chain extension based on two-stage anaerobic fermentation, the culture substrate includes a mixture of ammonium chloride, magnesium sulfate heptahydrate, potassium dihydrogen phosphate, dipotassium hydrogen phosphate, sodium chloride, vitamin solution, and trace element solution;
a concentration of the ammonium chloride in the second anaerobic fermentation mixture is 0.23-0.27 g/L;
a concentration of the magnesium sulfate heptahydrate in the second anaerobic fermentation mixture is 0.1-0.4 g/L;
a concentration of the potassium dihydrogen phosphate in the second anaerobic fermentation mixture is 0.21-0.25 g/L;
a concentration of the dipotassium hydrogen phosphate in the second anaerobic fermentation mixture is 0.29-0.33 g/L;
a concentration of the sodium chloride in the second anaerobic fermentation mixture is 0.6-1 g/L;
a concentration of the vitamin solution in the second anaerobic fermentation mixture is 1-3 mL/L; and
a concentration of the trace element solution in the second anaerobic fermentation mixture is 1-3 mL/L.

Preferably, in the method for synthesizing caproic acid by carbon chain extension based on two-stage anaerobic fermentation, the first methane inhibitor comprises sodium 2-bromoethanesulfonate; and
a concentration of the first methane inhibitor in the second anaerobic fermentation mixture is 8-12 g/L.

Preferably, in the method for synthesizing caproic acid by carbon chain extension based on two-stage anaerobic fermentation, the second anaerobic fermentation conditions include: an initial pH of 4-8, a temperature of 20-50 °C, a rotation speed of 100-200 rpm, and a reaction time of 2-14 days.

It may be known from the technical solutions that, compared with the prior art, the present invention has the following beneficial effects.
(1) The method disclosed by the present invention performs alkali pretreatment on concentrated sludge, so that the breakage of cells in the sludge (lysis of cell wall structure) and the dissolution of extracellular polymers are promoted to a great extent, more protein is decomposed into amino acid under the action of protease during the carbon chain extension reaction, the construction of the sludge organic substrate is further optimized, a more sufficient organic substrate is provided for the subsequent synthesis of caproic acid in the carbon chain extension process, the synthesis conversion efficiency of the carbon chain extension functional flora is improved, the biological resource transformation of sludge is promoted to develop towards a more efficient direction, and an important theoretical practical basis is provided for achieving industrial production in the future.
(2) In the two-stage anaerobic fermentation solution for synthesizing caproic acid by carbon chain extension using short-term pre-fermented sludge as the substrate, the caproic acid yield in a two-stage experimental group under the action of caproic acid-producing bacteria is 156.07 mg COD/g VSS, which are 3.05 and 2.51 times that of the one-stage control group, respectively. Therefore, the effect of synthesizing caproic acid by carbon chain extension in two-stage anaerobic fermentation is much higher than that in one-stage anaerobic fermentation. Moreover, the acetic acid in the two-stage fermentation sludge experimental group reaches 124.11 mg COD/g VSS, which is 6.50 times that in the one-stage reactor, thereby providing more sufficient substrate for the subsequent anaerobic fermentation reaction. Compared with the one-stage anaerobic fermentation, the two-stage anaerobic fermentation has higher economic utilization and excellent environmental friendliness, the caproic acid produced by the two-stage anaerobic fermentation is far higher than that of the one-stage anaerobic fermentation, and the solution of the present invention greatly promotes the breakage of cells in sludge and the dissolution of extracellular polymer substances, which is of great significance for achieving the reduction, harmlessness and resource utilization of sludge.

### BRIEF DESCRIPTION OF DRAWINGS

To more clearly illustrate the technical solutions in the examples of the present invention or in the prior art, the drawings used in the description of the examples or the prior art are briefly introduced below.

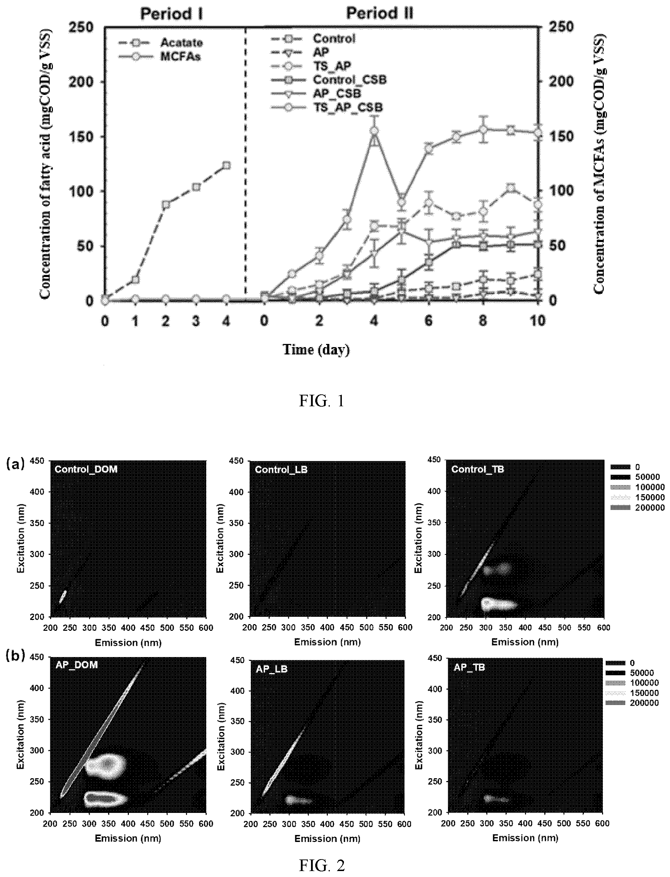

FIG. 1 is a diagram comparing the acid production results in the methods described in Example 1 and Comparative Examples 1 to 5; and
FIG. 2 is a diagram comparing three-dimensional fluorescence spectra of dissolved organic matter (DOM), loosely bound extracellular polymeric substances (LB-EPS) and tightly bound extracellular polymeric substances (TB-EPS) by the methods described in Example 1 and Comparative Example 1; wherein a is Comparative Example 1, Control_DOM in a is a control group of dissolved organic matter in Comparative Example 1, Control_LB in a is a control group of loosely bound extracellular polymeric substances in Comparative Example 1, and Control_TB in a is a control group of tightly bound extracellular polymeric substances in Comparative Example 1; b is Example 1, AP_DOM in b is an experimental group of alkali-pretreated dissolved organic matter in Example 1, AP_LB in b is an experimental group of alkali-pretreated loosely bound extracellular polymeric substances in Example 1, and AP_TB in b is an experimental group of alkali-pretreated tightly bound extracellular polymeric substances in Example 1.

### DETAILED DESCRIPTION OF EMBODIMENTS

The present invention provides a method for synthesizing caproic acid by carbon chain extension based on two-stage anaerobic fermentation, which includes the following steps:
performing alkali pretreatment on concentrated sludge; performing first anaerobic fermentation on the concentrated sludge subjected to alkali pretreatment to obtain a fermentation product; and
mixing the fermentation product, caproic acid-producing bacteria, an electron donor, a culture substrate and a first methane inhibitor, and performing second anaerobic fermentation to obtain the caproic acid.

In the present invention, the concentrated sludge is preferably obtained in the following manner: standing excess sludge, draining a supernatant, and concentrating; and sieving the concentrated excess sludge with a 40-mesh sieve to obtain the concentrated sludge.

In the present invention, the source of the excess sludge is preferably a secondary sedimentation tank of a sewage treatment plant.

In the present invention, the standing conditions include: a temperature is preferably 0-10 °C, further preferably 2-6 °C, and more preferably 4 °C; a standing time is preferably 10-24 h, further preferably 20-24 h, and more preferably 24 h.

In the present invention, a pH value of the concentrated sludge is preferably 7.33-7.37, further preferably 7.34-7.36, and more preferably 7.35; a total suspended solids concentration of the concentrated sludge is preferably 31.81-31.85 g/L, further preferably 31.82-31.84 g/L, and more preferably 31.83 g/L; and a volatile suspended solids concentration of the concentrated sludge is preferably 12.03-12.07 g/L, further preferably 12.04-12.06 g/L, and more preferably 12.05 g/L.

In the present invention, a reagent used in the alkali pretreatment is preferably NaOH mother liquor.

In the present invention, the NaOH mother liquor preferably includes sodium hydroxide and hydrochloric acid.

In the present invention, a concentration of the sodium hydroxide in the NaOH mother liquor is preferably 3-5 mol/L, further preferably 3.5-4.5 mol/L, and more preferably 4 mol/L.

In the present invention, a mass fraction of the hydrochloric acid in the NaOH mother liquor is preferably 10-14%, further preferably 10-12%, and more preferably 12%.

In the present invention, a mass ratio of the sodium hydroxide in the NaOH mother liquor to the total suspended solids in the concentrated sludge is preferably 0.05-0.1:1, further preferably 0.07-0.1:1, and more preferably 0.09:1.

In the present invention, the alkali pretreatment conditions include: a pH value is preferably 10-14, further preferably 11.9-12.1, and more preferably 12; a temperature is preferably 110-130 °C, further preferably 115-125 °C, and more preferably 120 °C; and an alkali pretreatment time is preferably 10-30 min, further preferably 15-25 min, and more preferably 20 min.

In the present invention, sterilization is performed during the alkali pretreatment.

In the present invention, nitrogen gas is introduced before the first anaerobic fermentation is performed to ensure an anaerobic environment. In the present invention, a time for introducing nitrogen is preferably 5-20 min, further preferably 8-12 min, and more preferably 10 min.

In the present invention, the first anaerobic fermentation conditions include: an initial pH value is preferably 5-7, further preferably 5.7-6.3, and more preferably 6; a temperature is preferably 30-40 °C, further preferably 30-35 °C, and more preferably 30 °C; a rotation speed is preferably 100-200 rpm, further preferably 110-130 rpm, and more preferably 120 rpm; and a reaction time is preferably 3-4 days, further preferably 3.5-4 days, and more preferably 4 days.

In the present invention, the caproic acid-producing bacteria include one or more of Clostridium_sensu_stricto (with a strain No. CICC 20464 and a Latin name of *Bacillus fusiformis*), Romboutsia (with a Latin genus of *Romboutsia,* a Latin species of lituseburensis, and an original No. YB101), and Terrisporobacter (with a strain No. CICC 6006 and a Latin name of *Bacillus ginsengihumi*), further preferably Clostridium_sensu_stricto or Romboutsia, and more preferably Clostridium_sensu_stricto.

In the present invention, the source of the caproic acid-producing bacteria is preferably: excess sludge from Shanxi Zhengyang Wastewater Purification Co., Ltd.

In the present invention, a mass ratio of the concentrated sludge to the caproic acid-producing bacteria is preferably 1-2:9-12, further preferably 1-2:9-10, and more preferably 1:9.

In the present invention, the caproic acid-producing bacteria are preferably acclimatized before use.

In the present invention, the acclimatization process preferably includes the following steps: mixing the caproic acid-producing bacteria, a chain extension substrate, a culture medium and a second methane inhibitor under an anaerobic condition for culturing.

In the present invention, the chain extension substrate preferably includes a mixture of sodium acetate and ethanol.

In the present invention, a use amount of the sodium acetate in the acclimatization process is preferably 4-6 g/L, further preferably 4.8-5.3 g/L, and more preferably 5 g/L.

In the present invention, a use amount of the ethanol in the acclimatization process is preferably 10-30 mL/L, further preferably 19-24 mL/L, and more preferably 20 mL/L.

In the present invention, the culture medium preferably includes: a mixture of ammonium dihydrogen phosphate, sodium bicarbonate, mineral solution, and vitamin solution.

In the present invention, a use amount of the diammonium phosphate in the acclimatization process is preferably 3.3-3.8 g/L, further preferably 3.4-3.7 g/L, and more preferably 3.6 g/L.

In the present invention, a use amount of the sodium bicarbonate in the acclimatization process is preferably 5.2-5.7 g/L, further preferably 5.3-5.6 g/L, and more preferably 5.4 g/L.

In the present invention, a use amount of the mineral solution in the acclimatization process is preferably 3-6 mL/L, further preferably 4-5 mL/L, and more preferably 5 mL/L.

In the present invention, a use amount of the vitamin solution in the acclimatization process is preferably 7-13 µL/L, further preferably 8-12 µL/L, and more preferably 10 µL/L.

In the present invention, the second methane inhibitor preferably includes sodium 2-bromoethanesulfonate.

In the present invention, a use amount of the second methane inhibitor in the acclimatization process is preferably 5-15 g/L, further preferably 8-12 g/L, and more preferably 10 g/L.

In the present invention, no nitrogen is preferably introduced during the culture process.

In the present invention, the culture conditions include: a pH value is preferably 4-8, further preferably 5-7, and more preferably 6; a temperature is preferably 20-50 °C, further preferably 30-40 °C, and more preferably 30 °C; a rotation speed is preferably 100-200 rpm, further preferably 110-150 rpm, and more preferably 120 rpm; a culture time is preferably 2-7 days, further preferably 5-7 days, and more preferably 7 days.

In the present invention, the electron donor preferably includes ethanol and/or lactic acid, further preferably ethanol.

In the present invention, a molar ratio of an electron acceptor to the electron donor in the fermentation product is preferably 1:3-5, further preferably 1:3-4, and more preferably 1:3.

In the present invention, the electron acceptor in the fermentation product is preferably acetic acid.

In the present invention, the culture substrate preferably includes a mixture of ammonium chloride, magnesium sulfate heptahydrate, potassium dihydrogen phosphate, dipotassium hydrogen phosphate, sodium chloride, vitamin solution, and trace element solution.

In the present invention, a concentration of the ammonium chloride in the second anaerobic fermentation mixture is preferably 0.23-0.27 g/L, further preferably 0.24-0.26 g/L, and more preferably 0.25 g/L.

In the present invention, a concentration of the magnesium sulfate heptahydrate in the second anaerobic fermentation mixture is preferably 0.1-0.4 g/L, further preferably 0.1-0.3 g/L, and more preferably 0.2 g/L.

In the present invention, a concentration of the potassium dihydrogen phosphate in the second anaerobic fermentation mixture is preferably 0.21-0.25 g/L, further preferably 0.22-0.24 g/L, and more preferably 0.23 g/L.

In the present invention, a concentration of the dipotassium hydrogen phosphate in the second anaerobic fermentation mixture is preferably 0.29-0.33 g/L, further preferably 0.3-0.32 g/L, and more preferably 0.31 g/L.

In the present invention, a concentration of the sodium chloride in the second anaerobic fermentation mixture is preferably 0.6-1 g/L, further preferably 0.7-0.9 g/L, and more preferably 0.8 g/L.

In the present invention, a concentration of the vitamin solution in the second anaerobic fermentation mixture is preferably 1-3 mL/L, further preferably 1-2 mL/L, and more preferably 1 mL/L.

In the present invention, a concentration of the trace element solution in the second anaerobic fermentation mixture is preferably 1-3 mL/L, further preferably 1-2 mL/L, and more preferably 1 mL/L.

In the present invention, the first methane inhibitor preferably includes sodium 2-bromoethanesulfonate.

In the present invention, a concentration of the first methane inhibitor in the second anaerobic fermentation mixture is preferably 8-12 g/L, further preferably 9-11 g/L, and more preferably 10 g/L.

In the present invention, nitrogen purging is performed before the second anaerobic fermentation. In the present invention, a nitrogen purge time is preferably 8-12 min, further preferably 9-11 min, and more preferably 10 min.

In the present invention, the second anaerobic fermentation condition include: an initial pH value is preferably 4-8, further preferably 5-7, and more preferably 6; a temperature is preferably 20-50 °C, further preferably 30-40 °C, and more preferably 30 °C; a rotation speed is preferably 100-200 rpm, further preferably 110-150 rpm, and more preferably 120 rpm; a reaction time is preferably 2-14 days, further preferably 10-14 days, and more preferably 14 days.

The technical solutions in the examples of the present invention will be clearly and completely described below. Apparently, the described examples are merely a part, rather than all of the examples of the present invention. Based on the examples of the present invention, all other examples obtained by those of ordinary skill in the art without creative efforts shall fall within the protection scope of the present invention.

### Example 1

This example provides a method for synthesizing caproic acid by carbon chain extension based on two-stage anaerobic fermentation, which is denoted as TS_AP_CSB and includes the following steps:
(1) The excess sludge was taken from a secondary sedimentation tank of a sewage treatment plant (from Shanxi Zhengyang Wastewater Purification Co., Ltd. on June 7, 2022), and left to stand naturally at 4 °C for 24 hours. The supernatant was discharged for concentration. After concentration, impurities in the sludge were removed by a 40-mesh sieve to obtain concentrated sludge with a pH value of 7.35, a total suspended solids concentration of 31.83 g/L, and a volatile suspended solids concentration of 12.05 g/L.
(2) 100 mL of caproic acid-producing bacteria (Clostridium_sensu_stricto, with a strain No. CICC 20464, a Latin name of *Bacillus fusiformis*, excess sludge from Shanxi Zhengyang Wastewater Purification Co., Ltd.) were taken in a proportion and inoculated in a 500 mL anaerobic fermentation bottle. Under anaerobic conditions, 5 g/L sodium acetate and 20 mL/L ethanol were used as chain extension substrates, and a culture medium was added. The culture medium components included 3.6 g/L ammonium dihydrogen phosphate, 5.4 g/L sodium bicarbonate, 5 mL/L mineral solution (0.10 g MnSO₄·H₂O, 0.12 g ZnSO₄·7H₂O, 0.07 g H₃BO₃, 0.04 g Na₂MoO₄·2H₂O, 0.02 g CuSO₄·5H₂, 0.04 g CoCl₂, and 1 L water) and 10 µL/L vitamin solution (20 mg biotin, 20 mg folic acid, 100 mg pyridoxine hydrochloride, 5.0 mg thiamine hydrochloride, 50 mg riboflavin, 50 mg niacin, 50 mg calcium pantothenate, 1 mg vitamin B12, 50 mg p-aminobenzoic acid, 50 mg lipoic acid, and 1 L distilled water). In addition, 10 g/L sodium 2-bromoethane sulfonate was added as a methanogenic inhibitor. The foregoing added substances were calculated based on the total amount during the acclimatization process. During the acclimatization process, the pH value was controlled at about 6.0, the temperature was controlled at 30 °C, the shaking speed of a shaker was 120 rpm, and the acclimatization time was 7 days.
(3) The concentrated sludge was subjected to alkali pretreatment, and a high-concentration NaOH mother liquor (the concentration of sodium hydroxide was 4 mol/L, and the mass fraction of hydrochloric acid was 12%) was added. The mass ratio of the sodium hydroxide in the NaOH mother liquor to the total suspended solids of the concentrated sludge was 0.09:1. The pH was adjusted to 12, and the sludge was treated at 120 °C for 20 min. During the treatment, the concentrated sludge was sterilized in a high-temperature sterilizer. The alkali-pretreated concentrated sludge was then placed in a fermentation bottle for anaerobic fermentation. Nitrogen was filled for 10 min to ensure the anaerobic environment. The fermentation bottle was placed in a shaker for culture. The initial pH value during fermentation was 6. The fermentation was performed at 30 °C and a shaking speed of 120 rpm for 4 days. The fermentation was stopped when the volatile fatty acid yield reached the highest level, and the fermentation product was obtained.
(4) The fermentation product was placed in a fermentation bottle, and the acclimatized caproic acid-producing bacteria were added. The mass ratio of the concentrated sludge to the caproic acid-producing bacteria was 1:9. The electron donor ethanol was added to the bottle at a molar ratio of electron acceptor acetic acid: electron donor=1:3, and a culture substrate was added. The culture substrate contained 0.25 g/L ammonium chloride, 0.2 g/L magnesium sulfate heptahydrate, 0.23 g/L potassium dihydrogen phosphate, 0.31 g/L dipotassium hydrogen phosphate, 0.8 g/L sodium chloride, 1 mL/L vitamin solution (20 mg biotin, 20 mg folic acid, 100 mg pyridoxine hydrochloride, 5.0 mg thiamine hydrochloride, 50 mg riboflavin, 50 mg niacin, 50 mg calcium pantothenate, 1 mg vitamin B12, 50 mg p-aminobenzoic acid, 50 mg lipoic acid and 1 L distilled water), and 1 mL/L trace element solution (0.10 g MnSO₄·H₂O, 0.12 g ZnSO₄·7H₂O, 0.07 g H₃BO₃, 0.04 g Na₂MoO₄·2H₂O, 0.02 g CuSO₄·5H₂O, 0.04 g CoCl₂ and 1 L water). 10 g/L sodium 2-bromoethane sulfonate was added to inhibit methane production. The culture medium and sodium 2-bromoethane sulfonate are calculated based on the total amount in the anaerobic fermentation process. The pH value was adjusted to 6.0. Finally, the bottle cap was tightened, and after purging with high-purity nitrogen for 10 min, the bottle was placed in a shaker for anaerobic fermentation. The fermentation was performed at 30 °C and a shaking speed of 120 rpm for 14 days until the caproic acid yield reached the maximum.

### Comparative Example 1

This comparative example provides a method for synthesizing caproic acid by carbon chain extension based on one-stage anaerobic fermentation of sludge without alkali pretreatment, which is denoted as Control and includes the following steps:
(1) The excess sludge was taken from a secondary sedimentation tank of a sewage treatment plant (from Shanxi Zhengyang Wastewater Purification Co., Ltd. on June 7, 2022), and left to stand naturally at 4 °C for 24 hours. The supernatant was discharged for concentration. After concentration, impurities in the sludge were removed by a 40-mesh sieve to obtain concentrated sludge with a pH value of 7.35, a total suspended solids concentration of 31.83 g/L, and a volatile suspended solids concentration of 12.05 g/L.
(2) The concentrated sludge was placed in a fermentation bottle, and a culture substrate was added. The culture substrate contained 0.25 g/L ammonium chloride, 0.2 g/L magnesium sulfate heptahydrate, 0.23 g/L potassium dihydrogen phosphate, 0.31 g/L dipotassium hydrogen phosphate, 0.8 g/L sodium chloride, 1 mL/L vitamin solution (20 mg biotin, 20 mg folic acid, 100 mg pyridoxine hydrochloride, 5.0 mg thiamine hydrochloride, 50 mg riboflavin, 50 mg niacin, 50 mg calcium pantothenate, 1 mg vitamin B12, 50 mg p-aminobenzoic acid, 50 mg lipoic acid and 1 L distilled water), and 1 mL/L trace element solution (0.10 g MnSO₄·H₂O, 0.12 g ZnSO₄·7H₂O, 0.07 g H₃BO₃, 0.04 g Na₂MoO₄·2H₂O, 0.02 g CuSO₄·5H₂O, 0.04 g CoCl₂ and 1 L water). 10 g/L sodium 2-bromoethane sulfonate was added to inhibit methane production. The culture medium and sodium 2-bromoethane sulfonate are calculated based on the total amount in the anaerobic fermentation process. The pH value was adjusted to 6.0. Finally, the bottle cap was tightened, and after purging with high-purity nitrogen for 10 min, the bottle was placed in a shaker for anaerobic fermentation. The fermentation was performed at 30 °C and a shaking speed of 120 rpm for 4 days until the caproic acid yield reached the maximum.

### Comparative Example 2

This comparative example provides a method for synthesizing caproic acid by carbon chain extension based on one-stage anaerobic fermentation of sludge without alkali pretreatment and addition of caproic acid-producing bacteria, which is denoted as Control_CSB and includes the following steps:
(1) The excess sludge was taken from a secondary sedimentation tank of a sewage treatment plant (from Shanxi Zhengyang Wastewater Purification Co., Ltd. on June 7, 2022), and left to stand naturally at 4 °C for 24 hours. The supernatant was discharged for concentration. After concentration, impurities in the sludge were removed by a 40-mesh sieve to obtain concentrated sludge with a pH value of 7.35, a total suspended solids concentration of 31.83 g/L, and a volatile suspended solids concentration of 12.05 g/L.
(2) 100 mL of caproic acid-producing bacteria (Clostridium_sensu_stricto, with a strain No. CICC 20464, a Latin name of *Bacillus fusiformis*, excess sludge from Shanxi Zhengyang Wastewater Purification Co., Ltd.) were taken in a proportion and inoculated in a 500 mL anaerobic fermentation bottle. Under anaerobic conditions, 5 g/L sodium acetate and 20 mL/L ethanol were used as chain extension substrates, and a culture medium was added. The culture medium components included 3.6 g/L ammonium dihydrogen phosphate, 5.4 g/L sodium bicarbonate, 5 mL/L mineral solution (0.10 g MnSO₄·H₂O, 0.12 g ZnSO₄·7H₂O, 0.07 g H₃BO₃, 0.04 g Na₂MoO₄·2H₂O, 0.02 g CuSO₄·5H₂, 0.04 g CoCl₂, and 1 L water) and 10 µL/L vitamin solution (20 mg biotin, 20 mg folic acid, 100 mg pyridoxine hydrochloride, 5.0 mg thiamine hydrochloride, 50 mg riboflavin, 50 mg niacin, 50 mg calcium pantothenate, 1 mg vitamin B12, 50 mg p-aminobenzoic acid, 50 mg lipoic acid, and 1 L distilled water). In addition, 10 g/L sodium 2-bromoethane sulfonate was added as a methanogenic inhibitor. The foregoing added substances were calculated based on the total amount during the acclimatization process. During the acclimatization process, the pH value was controlled at about 6.0, the temperature was controlled at 30 °C, the shaking speed of a shaker was 120 rpm, and the acclimatization time was 7 days.
(3) The concentrated sludge was placed in a fermentation bottle, and acclimatized caproic acid-producing bacteria were added. The ratio of concentrated sludge to caproic acid-producing bacteria was 1:9. The culture substrate was added. The culture substrate contained 0.25 g/L ammonium chloride, 0.2 g/L magnesium sulfate heptahydrate, 0.23 g/L potassium dihydrogen phosphate, 0.31 g/L dipotassium hydrogen phosphate, 0.8 g/L sodium chloride, 1 mL/L vitamin solution (20 mg biotin, 20 mg folic acid, 100 mg pyridoxine hydrochloride, 5.0 mg thiamine hydrochloride, 50 mg riboflavin, 50 mg niacin, 50 mg calcium pantothenate, 1 mg vitamin B12, 50 mg p-aminobenzoic acid, 50 mg lipoic acid and 1 L distilled water), and 1 mL/L trace element solution (0.10 g MnSO₄·H₂O, 0.12 g ZnSO₄·7H₂O, 0.07 g H₃BO₃, 0.04 g Na₂MoO₄·2H₂O, 0.02 g CuSO₄·5H₂O, 0.04 g CoCl₂ and 1 L water). 10 g/L sodium 2-bromoethane sulfonate was added to inhibit methane production. The culture medium and sodium 2-bromoethane sulfonate are calculated based on the total amount in the anaerobic fermentation process. The pH value was adjusted to 6.0. Finally, the bottle cap was tightened, and after purging with high-purity nitrogen for 10 min, the bottle was placed in a shaker for anaerobic fermentation. The fermentation was performed at 30 °C and a shaking speed of 120 rpm for 14 days until the caproic acid yield reached the maximum.

### Comparative Example 3

This comparative example provides a method for synthesizing caproic acid by carbon chain extension based on one-stage anaerobic fermentation of sludge subjected to alkali pretreatment, which is denoted as AP and includes the following steps:
(1) The excess sludge was taken from a secondary sedimentation tank of a sewage treatment plant (from Shanxi Zhengyang Wastewater Purification Co., Ltd. on June 7, 2022), and left to stand naturally at 4 °C for 24 hours. The supernatant was discharged for concentration. After concentration, impurities in the sludge were removed by a 40-mesh sieve to obtain concentrated sludge with a pH value of 7.35, a total suspended solids concentration of 31.83 g/L, and a volatile suspended solids concentration of 12.05 g/L.
(2) The concentrated sludge was subjected to alkali pretreatment, and a high-concentration NaOH mother liquor (the concentration of sodium hydroxide was 4 mol/L, and the mass fraction of hydrochloric acid was 12%) was added. The mass ratio of the sodium hydroxide in the NaOH mother liquor to the total suspended solids of the concentrated sludge was 0.09:1. The pH was adjusted to 12, and the sludge was treated at 120 °C for 20 min. During the treatment, the concentrated sludge was sterilized in a high-temperature sterilizer.
(3) The concentrated sludge subjected to alkali pretreatment was placed in a fermentation bottle, and a culture substrate was added. The nutrients contained 0.25 g/L ammonium chloride, 0.2 g/L magnesium sulfate heptahydrate, 0.23 g/L potassium dihydrogen phosphate, 0.31 g/L dipotassium hydrogen phosphate, 0.8 g/L sodium chloride, 1 mL/L vitamin solution (20 mg biotin, 20 mg folic acid, 100 mg pyridoxine hydrochloride, 5.0 mg thiamine hydrochloride, 50 mg riboflavin, 50 mg niacin, 50 mg calcium pantothenate, 1 mg vitamin B12, 50 mg *p*-aminobenzoic acid, 50 mg lipoic acid and 1 L distilled water), and 1 mL/L trace element solution (0.10 g MnSO₄·H₂O, 0.12 g ZnSO₄·7H₂O, 0.07 g H₃BO₃, 0.04 g Na₂MoO₄·2H₂O, 0.02 g CuSO₄·5H₂O, 0.04 g CoCl₂ and 1 L water). 10 g/L sodium 2-bromoethane sulfonate was added to inhibit methane production. The culture medium and sodium 2-bromoethane sulfonate are calculated based on the total amount in the anaerobic fermentation process. The pH value was adjusted to 6.0. Finally, the bottle cap was tightened, and after purging with high-purity nitrogen for 10 min, the bottle was placed in a shaker for anaerobic fermentation. The fermentation was performed at 30 °C and a shaking speed of 120 rpm for 14 days until the caproic acid yield reached the maximum.

### Comparative Example 4

This comparative example provides a method for synthesizing caproic acid by carbon chain extension based on one-stage anaerobic fermentation of sludge subjected to alkali pretreatment and addition of caproic acid-producing bacteria, which is denoted as AP_CSB and includes the following steps:
(1) The excess sludge was taken from a secondary sedimentation tank of a sewage treatment plant (from Shanxi Zhengyang Wastewater Purification Co., Ltd. on June 7, 2022), and left to stand naturally at 4 °C for 24 hours. The supernatant was discharged for concentration. After concentration, impurities in the sludge were removed by a 40-mesh sieve to obtain concentrated sludge with a pH value of 7.35, a total suspended solids concentration of 31.83 g/L, and a volatile suspended solids concentration of 12.05 g/L.
(2) 100 mL of caproic acid-producing bacteria (Clostridium_sensu_stricto, with a strain No. CICC 20464, a Latin name of *Bacillus fusiformis*, excess sludge from Shanxi Zhengyang Wastewater Purification Co., Ltd.) were taken in a proportion and inoculated in a 500 mL anaerobic fermentation bottle. Under anaerobic conditions, 5 g/L sodium acetate and 20 mL/L ethanol were used as chain extension substrates, and a culture medium was added. The culture medium components included 3.6 g/L ammonium dihydrogen phosphate, 5.4 g/L sodium bicarbonate, 5 mL/L mineral solution (0.10 g MnSO₄·H₂O, 0.12 g ZnSO₄·7H₂O, 0.07 g H₃BO₃, 0.04 g Na₂MoO₄·2H₂O, 0.02 g CuSO₄·5H₂, 0.04 g CoCl₂, and 1 L water) and 10 µL/L vitamin solution (20 mg biotin, 20 mg folic acid, 100 mg pyridoxine hydrochloride, 5.0 mg thiamine hydrochloride, 50 mg riboflavin, 50 mg niacin, 50 mg calcium pantothenate, 1 mg vitamin B12, 50 mg p-aminobenzoic acid, 50 mg lipoic acid, and 1 L distilled water). In addition, 10 g/L sodium 2-bromoethane sulfonate was added as a methanogenic inhibitor. The foregoing added substances were calculated based on the total amount during the acclimatization process. During the acclimatization process, the pH value was controlled at about 6.0, the temperature was controlled at 30 °C, the shaking speed of a shaker was 120 rpm, and the acclimatization time was 7 days.
(3) The concentrated sludge was subjected to alkali pretreatment, and a high-concentration NaOH mother liquor (the concentration of sodium hydroxide was 4 mol/L, and the mass fraction of hydrochloric acid was 12%) was added. The mass ratio of the sodium hydroxide in the NaOH mother liquor to the total suspended solids of the concentrated sludge was 0.09:1. The pH was adjusted to 12, and the sludge was treated at 120 °C for 20 min. During the treatment, the concentrated sludge was sterilized in a high-temperature sterilizer.
(4) The concentrated sludge subjected to alkali pretreatment was placed in a fermentation bottle, and acclimatized caproic acid-producing bacteria were added. The mass ratio of the concentrated sludge subjected to alkali pretreatment to caproic acid-producing bacteria was 1:9. The culture substrate was added. The culture substrate contained 0.25 g/L ammonium chloride, 0.2 g/L magnesium sulfate heptahydrate, 0.23 g/L potassium dihydrogen phosphate, 0.31 g/L dipotassium hydrogen phosphate, 0.8 g/L sodium chloride, 1 mL/L vitamin solution (20 mg biotin, 20 mg folic acid, 100 mg pyridoxine hydrochloride, 5.0 mg thiamine hydrochloride, 50 mg riboflavin, 50 mg niacin, 50 mg calcium pantothenate, 1 mg vitamin B12, 50 mg *p*-aminobenzoic acid, 50 mg lipoic acid and 1 L distilled water), and 1 mL/L trace element solution (0.10 g MnSO₄·H₂O, 0.12 g ZnSO₄·7H₂O, 0.07 g H₃BO₃, 0.04 g Na₂MoO₄·2H₂O, 0.02 g CuSO₄·5H₂O, 0.04 g CoCl₂ and 1 L water). 10 g/L sodium 2-bromoethane sulfonate was added to inhibit methane production. The culture medium and sodium 2-bromoethane sulfonate are calculated based on the total amount in the anaerobic fermentation process. The pH value was adjusted to 6.0. Finally, the bottle cap was tightened, and after purging with high-purity nitrogen for 10 min, the bottle was placed in a shaker for anaerobic fermentation. The fermentation was performed at 30 °C and a shaking speed of 120 rpm for 14 days until the caproic acid yield reached the maximum.

### Comparative Example 5

This comparative example provides a method for synthesizing caproic acid by carbon chain extension based on two-stage anaerobic fermentation of sludge subjected to alkali pretreatment, which is denoted as TS_AP and includes the following steps:
(1) The excess sludge was taken from a secondary sedimentation tank of a sewage treatment plant (from Shanxi Zhengyang Wastewater Purification Co., Ltd. on June 7, 2022), and left to stand naturally at 4 °C for 24 hours. The supernatant was discharged for concentration. After concentration, impurities in the sludge were removed by a 40-mesh sieve to obtain concentrated sludge with a pH value of 7.35, a total suspended solids concentration of 31.83 g/L, and a volatile suspended solids concentration of 12.05 g/L.
(2) The concentrated sludge was subjected to alkali pretreatment, and a high-concentration NaOH mother liquor (the concentration of sodium hydroxide was 4 mol/L, and the mass fraction of hydrochloric acid was 12%) was added. The mass ratio of the sodium hydroxide in the NaOH mother liquor to the total suspended solids of the concentrated sludge was 0.09:1. The pH was adjusted to 12, and the sludge was treated at 120 °C for 20 min. During the treatment, the concentrated sludge was sterilized in a high-temperature sterilizer. The concentrated sludge was then placed in a fermentation bottle for anaerobic fermentation. Nitrogen was filled for 10 min to ensure the anaerobic environment. The fermentation bottle was placed in a shaker for culture. The initial pH value during fermentation was 6. The fermentation was performed at 30 °C and a shaking speed of 120 rpm for 4 days. The fermentation was stopped when the volatile fatty acid yield reached the highest level, and the fermentation product was obtained.
(3) The fermentation product was placed in a fermentation bottle. The electron donor ethanol was added to the bottle at a molar ratio of electron acceptor acetic acid: electron donor=1:3, and a culture substrate was added. The culture substrate contained 0.25 g/L ammonium chloride, 0.2 g/L magnesium sulfate heptahydrate, 0.23 g/L potassium dihydrogen phosphate, 0.31 g/L dipotassium hydrogen phosphate, 0.8 g/L sodium chloride, 1 mL/L vitamin solution (20 mg biotin, 20 mg folic acid, 100 mg pyridoxine hydrochloride, 5.0 mg thiamine hydrochloride, 50 mg riboflavin, 50 mg niacin, 50 mg calcium pantothenate, 1 mg vitamin B12, 50 mg p-aminobenzoic acid, 50 mg lipoic acid and 1 L distilled water), and 1 mL/L trace element solution (0.10 g MnSO₄·H₂O, 0.12 g ZnSO₄·7H₂O, 0.07 g H₃BO₃, 0.04 g Na₂MoO₄·2H₂O, 0.02 g CuSO₄·5H₂O, 0.04 g CoCl₂ and 1 L water). 10 g/L sodium 2-bromoethane sulfonate was added to inhibit methane production. The culture medium and sodium 2-bromoethane sulfonate are calculated based on the total amount in the anaerobic fermentation process. The pH value was adjusted to 6.0. Finally, the bottle cap was tightened, and after purging with high-purity nitrogen for 10 min, the bottle was placed in a shaker for anaerobic fermentation. The fermentation was performed at 30 °C and a shaking speed of 120 rpm for 14 days until the caproic acid yield reached the maximum.

After the sludge was pretreated, one-stage and two-stage fermentations (AP, AP_CSB, TS_AP and TS_AP_CSB) were performed, and blank control groups (Control and Control_CSB) were set for carbon chain extension to synthesize caproic acid. The changes in the caproic acid yield are shown in FIG. 1. It can be seen from FIG. 1 that, under the one-stage and two-stage fermentation conditions, the highest caproic acid yield was Control (23.70 mg COD/g VSS), AP (8.21 mg COD/g VSS), TS_AP (102.38 mg COD/g VSS), Control_CSB (51.12 mg COD/g VSS), AP_CSB (62.92 mg COD/g VSS), and TS_AP_CSB (156.07 mg COD/g VSS). The caproic acid yield of TS_AP was 1.25 and 3.60 times that of the Control and AP groups, respectively, and the caproic acid yield of TS_AP_CSB group was 3.05 and 1.52 times that of the Control_CSB and AP_CSB groups, respectively. The effect of synthesizing caproic acid by two-stage anaerobic fermentation is significantly better than that by one-stage anaerobic fermentation. Both Control and AP groups produced a small amount of caproic acid, which indicated that the sludge microbial community metabolized the substrate to a certain degree to synthesize a small amount of caproic acid, wherein the concentration of the caproic acid in the AP group was low, which might be due to the effect of alkali pretreatment on the remaining high-temperature spore-resistant bacteria, resulting in the lack of bacteria suitable for carbon chain extension in the AP group. In addition, although the two-stage sludge pretreatment control group (TS_AP) lacked sufficient carbon chain extension bacteria, the Period I provided sufficient substrates, and the microbial community could use short-chain fatty acids such as acetic acid produced in the pre-fermentation stage to produce more caproic acid than the other two groups of sludge bacterial metabolites (Control and AP). The caproic acid yield in the TS_AP_CSB and TS_AP groups was the highest at the end, which clearly showed that the two-stage anaerobic fermentation was more conducive to the carbon chain extension reaction, promoted the reaction process, and alleviated the reaction lag caused by the lack of short-chain fatty acids in the one-stage anaerobic fermentation system.

The fluorescence spectra comparison of the release of extracellular polymers from the original sludge and alkali pretreatment (AP) are shown in FIG. 2. The fluorescence characteristics stimulated by dissolved organic matter in the sample may be divided into five regions. The focus is on biodegradable organic matter in Zone I (i.e., tyrosine proteins, Ex/Em 200-250/200-330) and Zone IV (i.e., soluble microbial by-product substances, Ex/Em 250-280/200-380), while organic matter in Zone II (i.e., tryptophan substances, Ex/Em 200-250/330-380), Zone III (i.e., fulvic acid substances, Ex/Em 200-250/380-500) and Zone V (i.e., humic acid substances, Ex/Em 250-400/380-500) are generally considered to be non-biodegradable materials. It may be seen that the alkali pretreatment effectively destroyed the structure of the extracellular polymer substance, causing the fluorescence intensity of the outermost soluble organic matter layer of the extracellular polymer substance to increase significantly. This indicates that alkali pretreatment may effectively break the sludge cell walls, release more soluble organic matter, provide more available organic matter for microorganisms, increase the concentration of acetic acid, and thus provide a more sufficient organic substrate for the subsequent synthesis of medium-chain fatty acids.

In conclusion, the present invention discloses a method for synthesizing caproic acid by carbon chain extension based on two-stage anaerobic fermentation. Based on the fact that the acid production of anaerobic fermentation of excess sludge is limited to the hydrolysis stage of releasing macromolecular organic matter wrapped in cell walls and extracellular polymers, alkali pretreatment and two-stage pre-fermentation and the addition of caproic acid-producing bacteria were proposed. The two-stage anaerobic fermentation is more conducive to the carbon chain extension reaction and produces a promotion process. Different from a one-stage anaerobic fermentation system that features a slow reaction process due to the lack of short-chain fatty acids, the two-stage anaerobic fermentation process generates more caproic acid. In addition, the alkali pretreatment effectively destroys the structure of the extracellular polymer substance, which indicates that the alkali pretreatment can release more dissolved organic matter, increase acetic acid concentration, provide more organic substrate, and thus increase the yield of caproic acid.

The above descriptions are only preferred examples of the present invention. It should be noted that those of ordinary skill in the art can also make several improvements and modifications without departing from the principle of the present invention, and such improvements and modifications shall fall within the protection scope of the present invention.

## Claims

1. A method for synthesizing caproic acid by carbon chain extension based on two-stage anaerobic fermentation, comprising the following steps:
performing alkali pretreatment on concentrated sludge; performing first anaerobic fermentation on the concentrated sludge subjected to alkali pretreatment to obtain a fermentation product; and
mixing the fermentation product, caproic acid-producing bacteria, an electron donor, a culture substrate and a first methane inhibitor, and performing second anaerobic fermentation to obtain the caproic acid.

2. The method for synthesizing caproic acid by carbon chain extension based on two-stage anaerobic fermentation according to claim 1, wherein a pH value of the concentrated sludge is 7.33-7.37, a total suspended solids concentration of the concentrated sludge is 31.81-31.85 g/L, and a volatile suspended solids concentration of the concentrated sludge is 12.03-12.07 g/L.

3. The method for synthesizing caproic acid by carbon chain extension based on two-stage anaerobic fermentation according to claim 1 or 2, wherein the alkali pretreatment conditions comprise: a pH value of 10-14, a temperature of 110-130 °C, and a reaction time of 10-30 min.

4. The method for synthesizing caproic acid by carbon chain extension based on two-stage anaerobic fermentation according to claim 1, wherein the first anaerobic fermentation conditions comprise: an initial pH value of 5-7, a temperature of 30-40 °C, a rotation speed of 100-200 rpm, and a reaction time of 3-4 days.

5. The method for synthesizing caproic acid by carbon chain extension based on two-stage anaerobic fermentation according to claim 1, wherein the caproic acid-producing bacteria comprise one or more of Clostridium_sensu_stricto, Romboutsia, and Terrisporobacter; and
a mass ratio of the concentrated sludge to the caproic acid-producing bacteria is 1-2:9-12.

6. The method for synthesizing caproic acid by carbon chain extension based on two-stage anaerobic fermentation according to claim 1 or 5, wherein the caproic acid-producing bacteria are acclimatized before use;
the acclimatization process comprises the following steps: mixing the caproic acid-producing bacteria, a chain extension substrate, a culture medium and a second methane inhibitor under an anaerobic condition for culturing; and
the culture conditions comprise: a pH value of 4-8, a temperature of 20-50 °C, a rotation speed of 100-200 rpm, and a culture time of 2-7 days.

7. The method for synthesizing caproic acid by carbon chain extension based on two-stage anaerobic fermentation according to claim 1, wherein the electron donor comprises ethanol and/or lactic acid; and
a molar ratio of an electron acceptor to the electron donor in the fermentation product is 1:3-5.

8. The method for synthesizing caproic acid by carbon chain extension based on two-stage anaerobic fermentation according to claim 1 or 7, wherein the culture substrate comprises a mixture of ammonium chloride, magnesium sulfate heptahydrate, potassium dihydrogen phosphate, dipotassium hydrogen phosphate, sodium chloride, vitamin solution, and trace element solution;
a concentration of the ammonium chloride in the second anaerobic fermentation mixture is 0.23-0.27 g/L;
a concentration of the magnesium sulfate heptahydrate in the second anaerobic fermentation mixture is 0.1-0.4 g/L;
a concentration of the potassium dihydrogen phosphate in the second anaerobic fermentation mixture is 0.21-0.25 g/L;
a concentration of the dipotassium hydrogen phosphate in the second anaerobic fermentation mixture is 0.29-0.33 g/L;
a concentration of the sodium chloride in the second anaerobic fermentation mixture is 0.6-1 g/L;
a concentration of the vitamin solution in the second anaerobic fermentation mixture is 1-3 mL/L; and
a concentration of the trace element solution in the second anaerobic fermentation mixture is 1-3 mL/L.

9. The method for synthesizing caproic acid by carbon chain extension based on two-stage anaerobic fermentation according to claim 8, wherein the first methane inhibitor comprises sodium 2-bromoethanesulfonate; and
a concentration of the first methane inhibitor in the second anaerobic fermentation mixture is 8-12 g/L.

10. The method for synthesizing caproic acid by carbon chain extension based on two-stage anaerobic fermentation according to claim 1, wherein the second anaerobic fermentation conditions comprise: an initial pH of 4-8, a temperature of 20-50 °C, a rotation speed of 100-200 rpm, and a reaction time of 2-14 days.
